# EUROPEAN PATENT APPLICATION

(11) **EP 3 705 064 A1**
(43) Date of publication of application: **09.09.2020**
(21) Application number: 17930583.4
(22) Date of filing: 24.11.2017
(51) Int. Cl.: A61B 17/15, A61B 34/10, B33Y 80/00

(54) **GUIDE FOR MANDIBULAR RESECTION SURGERY**

(30) Priority: 03.11.2017 KR 20170146001
(71) Applicant: Anymedi Inc., Songpa-gu Seoul 05510 (KR)
(72) Inventor: BAEK, Jung Hwan, Seoul 06280 (KR); KIM, Guk Bae, Seoul 02624 (KR); CHOI, Seung Hyun, Hanam-si Gyeonggi-do 12972 (KR); SONG, Hyun Kyung, Seoul 05783 (KR); KIM, Dong Rok, Busan 49415 (KR); LIM, Min Je, Goyang-si Gyeonggi-do 10550 (KR); KO, Kyung Hyun, Goyang-si Gyeonggi-do 10513 (KR); OH, Yoon Suk, Daejeon 34092 (KR); JEONG, Young Seop, Seoul 02458 (KR)
(74) Representative: Patentanwälte Bals & Vogel
(86) International application number: PCT/KR2017/013525
(87) International publication number: WO 2019/088341

(57) **Abstract**

The present disclosure relates to a guide for mandibular resection surgery, including, a guide plate having, at its lower side, a boundary surface that guides a resection pathway of a resection saw during a mandibular resection surgery; and a fixing portion that is formed to extend from the guide plate to be combined with a mandible or tooth, thereby fixing a position of the guide plate, wherein the guide plate is foldable.

## Description

### 1. Field

The present disclosure relates to a guide for mandibular resection surgery, and more particularly, to a guide for mandibular resection surgery that may be fixed to a mandible, to protect the surrounding tissues, including nerves, and accurately direct a resection line during the surgery of resecting part of the mandible.

### 2. Background

Mandibular resection surgery has been widely used in recent years for cosmetic purposes and correction. One method of the mandibular resection surgery being utilized is to fix a guide to a mandible to form a resection line, and then to resect along the resection line of the guide using a resection saw.

When mounting the guide onto a mandible, the soft tissue around the mandible is peeled off and the guide is inserted through the peeled surface and mounted onto the mandible. Here, conventionally, the shape of the guide was fixed, and thus it was difficult to insert the guide or approach the surgical site with the guide during surgery with only a minimal amount of incision. Further, since the guide was made of a hard material, very strict peeling was required when peeling off the tissue from the bone, and if not peeled off completely, the small soft tissue fragments caused a large error in attaching the entire guide to the human body. This made the overall surgery site to be lengthy, and increased the degree and surface area of the peeling, which do not meet patients' demand for minimal incision surgery, and also causing problems of increased surgical time for incision and suturing.

In addition, in order to increase the accuracy of the surgery, a guide that is designed optimally for the mandible must be stably fixed in an accurate position.

### SUMMARY

Therefore, a purpose of the present disclosure is to resolve the aforementioned problems of prior art, that is, to provide a guide for mandibular resection surgery that can be folded before being mounted onto a mandible and then unfolded afterwards so that the soft tissue around the mandible can be resected minimally, and that the guide is customized to the patient and designed optimally, and fixed stably in an accurate position.

The tasks that the present disclosure intends to resolve are not limited to the aforementioned tasks, and other tasks that are not mentioned herein will also be clearly understood by a person skilled in the art based on the following description.

The aforementioned purposes can be achieved by the guide for mandibular resection surgery that includes a guide plate having, at its lower side, a boundary surface that guides a resection pathway of a resection saw during a mandibular resection surgery; and a fixing portion that is formed to extend from the guide plate and combine with a mandible or tooth, thereby fixing a position of the guide plate, wherein the guide plate is foldable.

Here, the guide plate may be made of a soft material.

Here, the guide plate may include a folding portion where the guide plate is foldable, and the folding portion may be made of a soft material.

Here, the soft material may include rubber, polymer, or silicon.

Here, the guide plate may be formed in a porous tube structure.

Here, the guide plate may include a folding portion where the guide plate is foldable, and the folding portion may be formed in a porous tube structure.

Here, the porous tube structure may be arranged in rectangular, spherical, honeycomb shape, or in straight line longitudinally in one direction.

Here, the guide plate may include a first guide plate that extends in a longitudinal direction from one side of a front surface to a rear side of the mandible, with the boundary surface at its lower side guiding the resection pathway.

Here, the guide plate may further include a second guide plate, that is formed to extend from a middle portion of the first guide plate towards the tooth.

Here, on the front surface of the first guide plate, a hole may be formed, into which a neural tube, protruding from one side of the front surface of the mandible, may be inserted.

Here, one side of the hole may have an incised shape so as to be connected with an outer side of the first guide plate.

Here, a first fixing portion may be formed, that is made of a hard material, and that extends from a rear end of the first guide plate, and bent, so as to combine with a corner of a rear side of the mandible.

Here, a second fixing portion may be formed, that is made of a hard material, and that extends from one end of the second guide plate, and bent, so as to combine with the tooth.

Here, a third fixing portion may be formed, that is made of a hard material, and that extends from one end at a lower side of a middle portion of the first guide plate, and bent, so as to combine with a corner of a lower side of the mandible.

Here, on the third fixing portion, a slit groove may be formed, where the resection saw may be inserted, to form the resection pathway of the resection saw during the resection surgery.

Here, an inner side of the second guide plate may be formed to be dented towards the inside, in consideration of a thickness of gum.

Here, the second guide plate from which the second fixing portion extends and the third fixing portion may be arranged in one row in a vertical direction, and the second fixing portion, the second guide plate, the third fixing portion, and the middle portion of the first guide plate from which the third fixing portion extends, arranged in one row, may be formed of a hard material, integrally.

Here, a middle portion of a rear end of the first guide plate may be made of a hard material, to prevent deformation from occurring due to self-weight.

Here, a metal rod may be inserted into a middle portion of a rear end of the first guide plate to prevent deformation from occurring due to self-weight.

Here, a shape design of the guide plate may be customized to a patient in consideration of a shape of the mandible of the patient and distribution of a neural tube inside the mandible, based on 3-dimensional image information on the mandible of the patient, so as not to damage the neural tube during the mandibular resection surgery, and a shape design of the fixing portion may be customized to the patient and manufactured such that a position of the guide plate can be stably fixed according to the shape of the mandible of the patient.

Here, the guide for mandibular resection surgery may be manufactured integrally using a 3-dimensional printer.

According to the guide for mandibular resection surgery of the present disclosure described above, the guide can be folded, and therefore, there is an advantage that the surface area of the soft tissue around the mandible being cut in order to mount the guide onto the mandible can be minimized.

There is also an advantage that, in the major landmarks such as the mandible corner, lower alveolar neural tube or tooth, etc., by using the hardness and elasticity of the materials, the guide can be fixed to the mandible stably in an accurate position.

There is also an advantage that, as the guide is customized to the patient and manufactured based on 3-dimensional image information of the patient, the resection line can be directed accurately without damaging the neural tube inside the mandible during the resection surgery.

Further, there is an advantage that, by using the 3-dimensional image information of the patient, the shape of the guide can be optimally designed, and by using a 3-dimensional printer, the guide consisting of a plurality of materials can be manufactured continuously at one time.

### BRIEF DESCRIPTION OF THE DRAWINGS

FIG. 1 is a front side perspective view of a guide for mandibular resection surgery according to a first embodiment of the present disclosure.
FIG. 2 is a rear side perspective view of FIG. 1.
FIG. 3 is a view schematically illustrating a lower alveolar neural tube inside a mandible.
FIG. 4 is a view illustrating the guide for mandibular resection surgery of FIG. 1, mounted onto a mandible.
FIG. 5 is a photograph of an actual manufactured guide for mandibular resection surgery of FIG. 1, that is folded.
FIG. 6 is a photograph of the guide for mandibular resection surgery of FIG. 5, mounted onto a mandible model.
FIG. 7 is a front side perspective view illustrating a guide for mandibular resection surgery according to a second embodiment of the present disclosure.
FIG. 8 is a front side perspective view illustrating a guide for mandibular resection surgery according to a third embodiment of the present disclosure.
FIG. 9 is a side view of a second guide plate.
FIG. 10 is a front side perspective view illustrating a guide for mandibular resection surgery according to a fourth embodiment of the present disclosure.
FIG. 11 is a front side perspective view illustrating a guide for mandibular resection surgery according to a fifth embodiment of the present disclosure.

### DETAILED DESCRIPTION

Specific matters of the embodiments are included in the detailed description and the drawings.

The advantages and characteristics of the present disclosure, and the method for achieving those advantages and characteristics will be clarified with reference to the embodiments that will be explained hereinafter together with the drawings attached hereto. However, the present disclosure is not limited to the embodiments disclosed hereinafter, but may be implemented in various different forms, and the present embodiments are provided merely for the purpose of complete disclosure of the present disclosure, and for the purpose of informing a person skilled in the art of the complete scope of the present disclosure, and the present disclosure is to be defined only by the scope of the claims. Like reference numerals indicate like configurative elements throughout the entirety of the specification.

Hereinbelow, the present disclosure will be described with reference to the drawings attached to describe a guide for mandibular resection surgery according to the embodiments of the present disclosure.

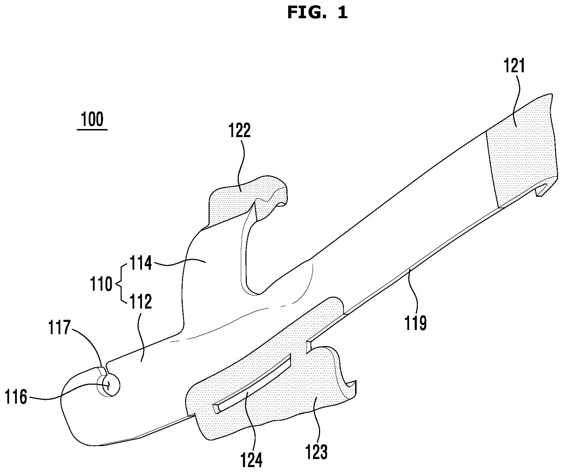

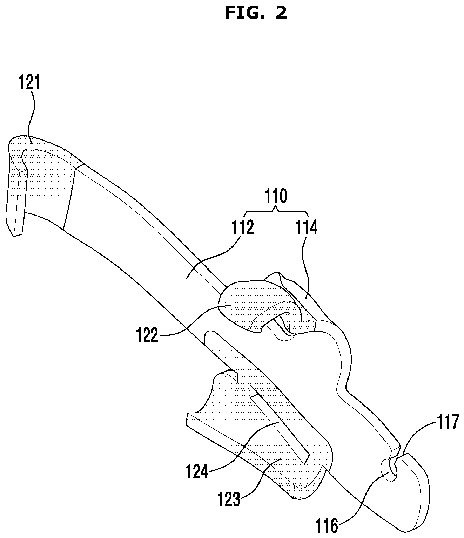

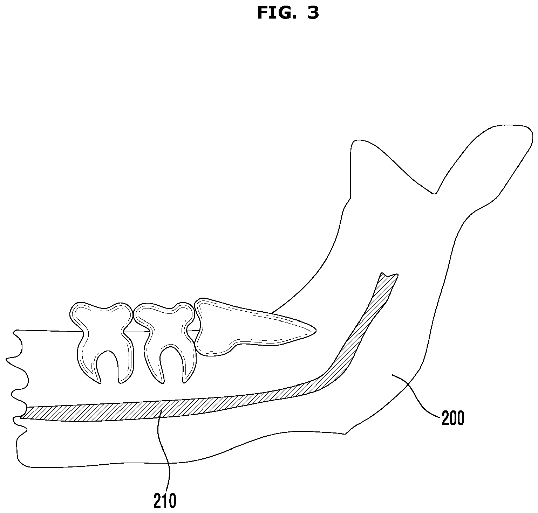

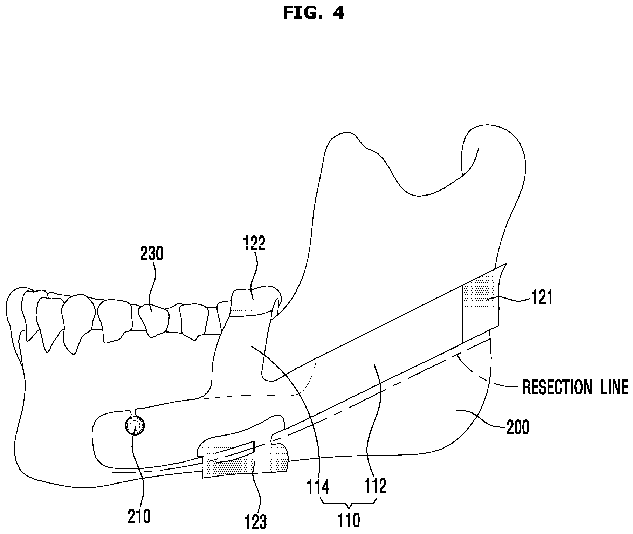

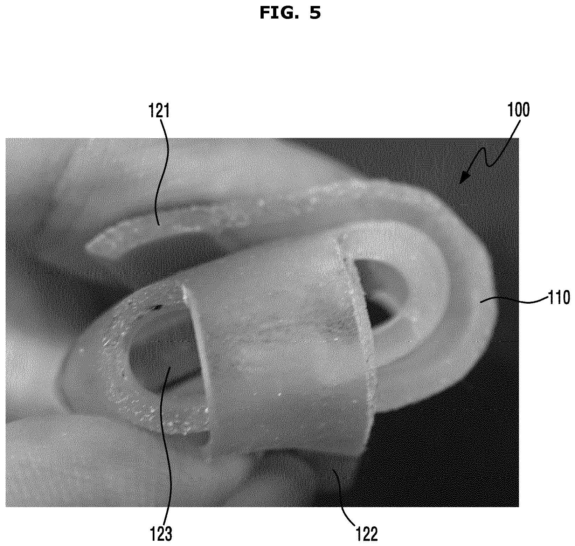

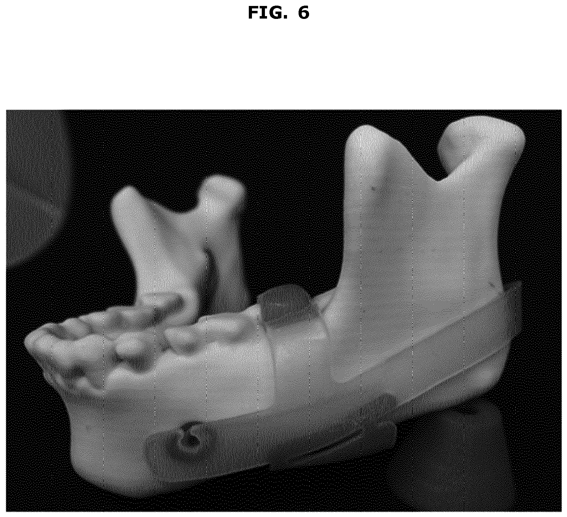

FIG. 1 is a front side perspective view of a guide for mandibular resection surgery according to a first embodiment of the present disclosure, FIG. 2 is a rear side perspective view of FIG. 1, FIG. 3 is a view schematically illustrating a lower alveolar neural tube inside a mandible, FIG. 4 is a view illustrating the guide for mandibular resection surgery of FIG. 1, mounted onto a mandible, FIG. 5 is a photograph of an actual manufactured guide for mandibular resection surgery of FIG. 1, that is folded, and FIG. 6 is a photograph of the guide for mandibular resection surgery of FIG. 5, mounted onto a mandible model.

The guide for mandibular resection guide according to the first embodiment of the present disclosure 100 may consist of a guide plate 110, and fixing portions 121, 122, 123.

The guide plate 100, when fixed to a mandible 200, enables a boundary surface 119, located at its lower side, to guide a resection pathway of a resection saw during a resection surgery of the mandible 200. Therefore, upon fixing the guide for mandibular resection surgery of the present disclosure 100 to the mandible 200, using the resection saw, it is possible to accurately resect a resection site along the boundary surface 119 located at the lower side of the guide plate 110.

The guide plate 110 may consist of a first guide plate 112 that is formed such that, when mounted onto the mandible 200, extends along a longitudinal direction from one side of a front surface of the mandible 200 to a rear side of the mandible 200, and that enables the boundary surface 119 at the lower side, to guide the resection pathway of the resection saw; and a second guide plate 114 that is formed to extend upwards from a middle portion of the first guide plate 112 towards a tooth 230.

Here, on a front surface of the first guide plate 112, a hole 116 may be formed, into which a lower alveolar neural tube 210, protruding from the side of the front surface of the mandible 200, may be inserted. As such, when fixing the guide for mandibular resection surgery 100 to the mandible 200, by inserting the neural tube 210 into the hole 116 formed on the first guide plate 112, it is possible to initially situate the guide 100.

Here, as illustrated in the drawings, at one side of the hole 116, an incision 117 may be formed, having an incised shape so as to be connected with an outer surface at an upper side of the first guide plate 112. As will be described below, the guide plate 110 may be made of a soft material, and thus, using the elasticity caused by the characteristics of the soft material and the incision 117, it is easy to insert the neural tube 210, protruding from the side of the mandible 200, into the hole 116, in a small space.

The hole 116 formed on the first guide plate 112 as described above, not only plays the role of initially positioning the guide 100 but, together with a first fixing portion 121 that will be described below, it also prevents the guide plate 110 from moving left and right.

In the present embodiment, the guide plate 110 may be made of a soft material, for example, a soft rubber, polymer or silicon, etc.

Therefore, in the present embodiment, since the guide plate 110 is made of a soft material, it can be folded as illustrated in FIG. 5. In order to fix the guide for mandibular resection surgery 100 to the mandible 200, the soft tissue around the mandible 200, which is the surgical site, has to be peeled off, and the guide 100 has to be fixed through this peeled site, but in the present embodiment, since the guide plate 110 can be folded, the surface area of the peeling can be reduced. Here, the guide 100, in a folded state, may be inserted through the peeled site, and then the folded portion may be unfolded, and then using the fixing portions 121, 122, 123, that will be described below, the guide 100 may be stably fixed in an accurate position in the mandible 200.

The fixing portions 121, 122, 123 are formed to extend from the guide plate 110, and the fixing portions 121, 122, 123 are combined with a corner of the mandible 200 or the tooth 230, to stably fix the guide plate 110, to the mandible 200. Here, in the present embodiment, the fixing portion may consist of the first fixing portion 121, the second fixing portion 122, or the third fixing portion 123, and it is desirable that the fixing portion is made of a hard material. Here, the hard material may be metal or ceramic, etc.

The first fixing portion 121 may be made of a hard material, and may be formed to extend from a rear end of the first guide plate 112 and bent, wherein the bent portion may be combined with a corner of a rear side of the mandible 200, thereby fixing the guide plate 110. Here, the aforementioned hole 116 formed at the front end of the first guide plate 112, and the first fixing portion 121, may be combined with the neural tube 210 and the corner of the rear side of the mandible 200, respectively, thereby preventing the guide plate 110 from moving left and right.

The second fixing portion 122 may be made of a hard material, and may be formed to extend from one end of the second guide plate 114 and bent, wherein the bent portion may be combined with the tooth 230, thereby fixing the guide plate 110.

The third fixing portion 123 may be made of a hard material, and may be formed to extend from one end at a lower side of the middle portion of the first guide plate 112 and bent, wherein the bent portion may be combined with a corner of a lower side of the mandible 200, thereby fixing the guide plate 110. Here, the third fixing portion 123 and the second fixing portion 122 may be combined with the corner of the lower side of the mandible 200 and the tooth 230, respectively, thereby preventing the guide plate 110 from moving up and down.

Here, in order to prevent the resection pathway formed by the boundary surface 119 at the lower side of the first guide plate 112, from being blocked by the third fixing portion 123, on the third fixing portion 123, a slit groove 124 is formed so that the resection saw can be inserted therein, thereby allowing the resection pathway to be formed, together with the boundary surface 119 at the lower side of the first guide plate 112.

A manufacturing method of the guide for mandibular resection surgery according to the present embodiment 100 involves, first of all, obtaining 3-dimensional image information of the mandible 200 of the patient in a well-known method such as CT and MRI, and then based on this information, understanding the shape of the mandible 200 of the patient, and the distribution of tissues such as the lower alveolar neural tube 210, that is inside the mandible 200 as shown in FIG. 3, that should be protected during the resection surgery. Next, using a computer aided design software, the resection line is planned out in consideration of the shape of the mandible 200 of the patient and the margin with the neural tube 210 that should be protected, based on the image information of the patient, and accordingly, the shape of the guide plate 110 and the fixing portions 121, 122, 123 for fixing the guide plate 110 are finally designed. Next, using the finally designed design information, the guide for mandibular resection surgery 100, made of soft and hard materials, can be continuously manufactured integrally by a 3-dimensional printer.

The guide for mandibular resection surgery 100 manufactured in the aforementioned method and mounted onto a mandible model is shown in a photograph in FIG. 6.

The guide for mandibular resection surgery according to the present embodiment 100 described above is manufactured to be customized to the patient based on the image information of the patient, and therefore does not damage the neural tube 210 inside the mandible 200 during the resection surgery, and can accurately direct the resection line. Further, as the 3-dimensional printer is used, it is possible to continuously manufacture the guide plate 110 of a soft material and the fixing portions 121, 122, 123 of a hard material, integrally, without any additional connecting portion.

Next, to describe the resection surgery process where the aforementioned guide for mandibular resection surgery according to the present embodiment 100 is used, first of all, the soft tissue around the mandible 200 is peeled off. Next, since the guide 100 of the present disclosure can be folded as in FIG. 5, the guide 100, in a folded state, is inserted into the peeled surface. Next, while unfolding the folded guide 100, by hanging the hole 116 formed on the first guide plate 112, and the first fixing portion 121, the second fixing portion 122 and the third fixing portion 123, on the lower alveolar neural tube 210, corner of the rear side of the mandible 200, tooth 230, and corner of the lower side of the mandible 200, respectively, the position of the guide 100 is fixed. Here, using the elasticity of the guide plate 110 made of a soft material, and the fixing portions 121, 122, 123, made of a hard material, it is possible to stably fix the guide 100 manufactured to be customized to the patient based on the image information of the mandible 200 of the patient, in an accurate position.

Next, using the resection saw, the mandible 200 is resected along the boundary surface 110 at the lower side of the first guide plate 112, wherein, when the resection site reaches the third fixing portion 123, the resection saw is removed, and then inserted again through the slit groove 124 of the third fixing portion 123, to perform the resecting. When the primary resection is completed in the aforementioned method, the guide 100 that had been fixed to the mandible 200, is removed and folded again, to be removed outside the peeling surface. Next, a portion between the boundary surface 119 at the lower side of the first guide plate 112 and the slit groove 124 of the third fixing portion 123, that had not been resected, is finally resected, and then, after tidying up the surface of the mandible 200, the surgery is finished by suturing back the peeled surface.

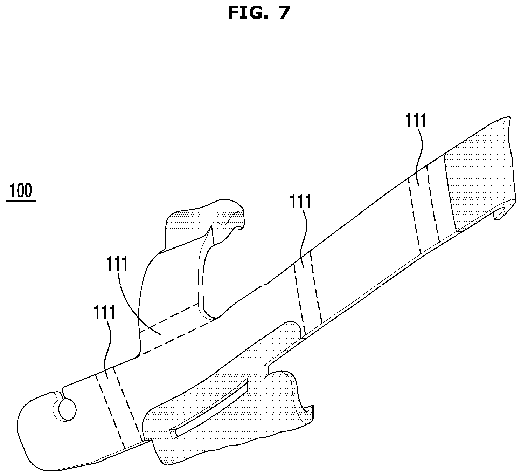

Hereinbelow, other embodiments of the present disclosure will be described with reference to FIGs. 7 to 11. The main focus of the description hereinbelow will be the differences from the embodiment described above with reference to FIGs. 1 to 6.

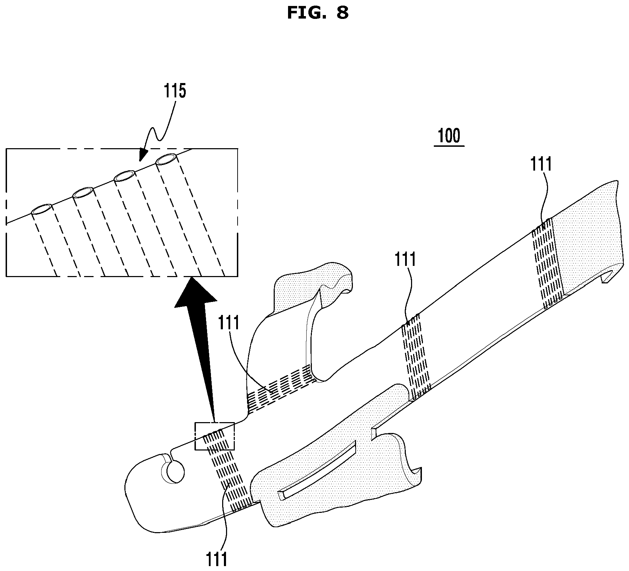

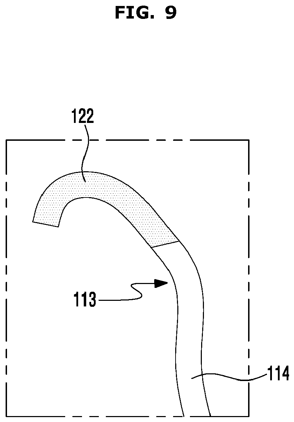

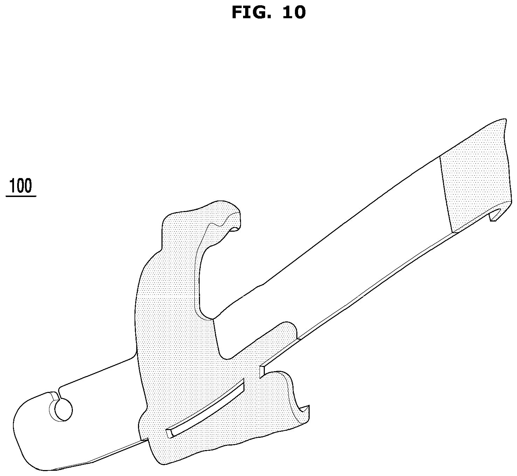

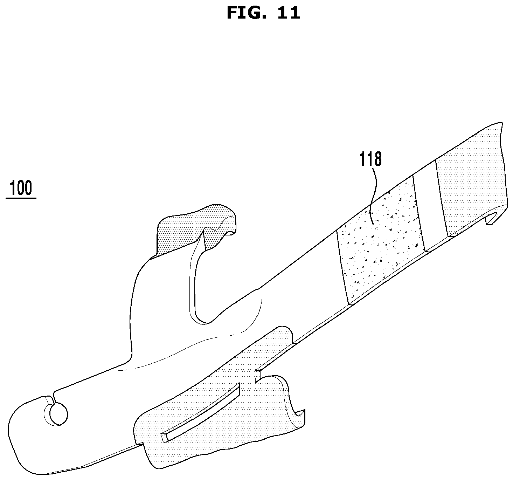

FIG. 7 is a front side perspective view illustrating the guide for mandibular resection surgery according to a second embodiment of the present disclosure, FIG. 8 is a front side perspective view illustrating the guide for mandibular resection surgery according to a third embodiment of the present disclosure, FIG. 9 is side view of the second guide plate, FIG. 10 is a front side perspective view illustrating the guide for mandibular resection surgery according to a fourth embodiment of the present disclosure, and FIG. 11 is a front side perspective view illustrating the guide for mandibular resection surgery according to a fifth embodiment of the present disclosure.

FIG. 7 is a side view of the guide for mandibular resection surgery according to the second embodiment of the present disclosure, and as illustrated in FIG. 7, on the guide plate 110, additional folding portion 111 may be formed on parts where the guide plate may be folded, and as this folding portion 111 is manufactured by a soft material, just as in the aforementioned embodiment, the guide plate 111 may be folded. Here, the rest of the portions besides the folding portion 111 of the guide plate 110 may be manufactured by a relatively harder material compared to the folding portion 111.

FIG. 8 is a side view of the guide for mandibular resection surgery according to a third embodiment of the present disclosure, and as illustrated in FIG. 8, on the guide plate 110, additional folding portion 111 may be formed on parts where the guide plate 110 may be folded, and the folding portion 111 is formed in a porous tube structure 115, and thus due to such structural characteristics, just as in the aforementioned embodiment, the guide plate 110 may be folded at the folding portions 111. The drawing illustrates a shape where the porous tube structure is arranged longitudinally in one direction, but it may be arranged in other various shapes instead, such as spheres, rectangles, or honeycombs, etc.

In FIG. 8, the folding portion 111 is formed in a predetermined area in the guide plate 110 to have the porous tube structure 115, but the entire guide plate 110 may of course be formed in the porous tube structure 115.

FIGs. 9 to 10 are views for describing the guide for mandibular resection surgery according to a fourth embodiment of the present disclosure, and just as in FIG. 9 that illustrates a side view of the second guide plate 114, in the present embodiment, when designing the shape of the second guide plate 114, the inner side portion that contacts the gum, is formed to be internally dented 113, in consideration of the thickness of the gum. As aforementioned, when designing the guide plate 110 using the 3-dimensional image information of the patient, the designing is based on the shape of the mandible 200, and in the present embodiment, the shape is designed in consideration of the thickness of the gum protruding from the bone underneath the tooth, and therefore, a more optimized shape design can be performed in accordance with the mounting site.

By designing the shape such that the inner surface of the second guide plate 114 is dented in consideration of the thickness of the gum as described above, it is possible to integrally form the second fixing portion 122, the second guide plate 114, the third fixing portion 123 and the middle portion of the first guide plate 112 from which the third fixing portion 123 extends, arranged in one row as in FIG. 10, with a hard material.

FIG. 11 is a side view of the guide for mandibular resection surgery according to a fifth embodiment of the present disclosure, wherein the rear end of the first guide plate 112, that is formed to extend longitudinally towards the right side based on the portion from which the second guide plate 114 extends from the first guide plate 112, is formed to have a long length, and therefore, due to the self-weight, up and down deformation may occur. This may act as an interfering element in stably fixing the guide 100 to the accurate position of the mandible 200, and therefore in the present embodiment, by forming the middle portion 118 of the rear end of the first guide plate 112 separately with a hard material as in FIG. 11, it is possible to prevent the up and down deformation from occurring due to the self-weight. Here, supposing that the degree of hardness of the first fixing portion 121, the second fixing portion 122 and the third fixing portion 123 is 100, it is desirable to control the degree of hardness in the middle portion 118 of the rear end of the first plate to be in a range not higher than 100 (for example, 90∼100), so as to prevent the deformation from occurring due to the self-weight, and to allow a certain degree of bending, at the same time.

Further, although not illustrated herein, by forming a structure where a thin metal rod may be inserted by forming a hole in an inner side of the middle portion 118 of the rear end of the first guide plate 112 or a ring at an outer side of the middle portion 228 of the rear end of the first guide plate 112, and then inserting the metal rod, it is possible to enable the metal rod to prevent the deformation of the middle portion 118.

The scope of right of the present disclosure is not limited to the aforementioned embodiments but may be implemented as various forms of embodiments within the attached claims set. Any person having ordinary skill in the art to which the present invention pertains will understand that the present disclosure may be implemented in other specific forms without departing from the gist of the present invention as claimed in the claims set to a wide range that can be modified.

## Claims

1. A guide for mandibular resection surgery comprising:
a guide plate having, at its lower side, a boundary surface that guides a resection pathway of a resection saw during a mandibular resection surgery; and
a fixing portion that is formed to extend from the guide plate and combine with a mandible or tooth, thereby fixing a position of the guide plate,
wherein the guide plate is foldable.

2. The guide for mandibular resection surgery, according to claim 1,
wherein the guide plate is made of a soft material.

3. The guide for mandibular resection surgery, according to claim 1,
wherein the guide plate comprises a folding portion where the guide plate is foldable, and
the folding portion is made of a soft material.

4. The guide for mandibular resection surgery, according to claim 2 or claim 3,
wherein the soft material comprises rubber, polymer, or silicon.

5. The guide for mandibular resection surgery, according to claim 1,
wherein the guide plate is formed in a porous tube structure.

6. The guide for mandibular resection surgery, according to claim 1,
wherein the guide plate comprises a folding portion where the guide plate is foldable, and
the folding portion is formed in a porous tube structure.

7. The guide for mandibular resection surgery, according to claim 5 or claim 6,
wherein the porous tube structure is arranged in rectangular, spherical, honeycomb shape, or in straight line longitudinally in one direction.

8. The guide for mandibular resection surgery, according to claim 1,
wherein the guide plate comprises a first guide plate that extends in a longitudinal direction from one side of a front surface to a rear side of the mandible, with the boundary surface at its lower side guiding the resection pathway.

9. The guide for mandibular resection surgery, according to claim 8,
wherein the guide plate further comprises a second guide plate, that is formed to extend from a middle portion of the first guide plate towards the tooth.

10. The guide for mandibular resection surgery, according to claim 8,
wherein on the front surface of the first guide plate, a hole is formed, into which a neural tube, protruding from one side of the front surface of the mandible, may be inserted.

11. The guide for mandibular resection surgery, according to claim 10,
wherein one side of the hole has an incised shape so as to be connected with an outer side of the first guide plate.

12. The guide for mandibular resection surgery, according to claim 8,
wherein a first fixing portion is formed, that is made of a hard material, and that extends from a rear end of the first guide plate, and bent, so as to combine with a corner of a rear side of the mandible.

13. The guide for mandibular resection surgery, according to claim 9,
wherein a second fixing portion is formed, that is made of a hard material, and that extends from one end of the second guide plate, and bent, so as to combine with the tooth.

14. The guide for mandibular resection surgery, according to claim 8,
wherein a third fixing portion is formed, that is made of a hard material, and that extends from one end at a lower side of a middle portion of the first guide plate, and bent, so as to combine with a corner of a lower side of the mandible.

15. The guide for mandibular resection surgery, according to claim 14,
wherein on the third fixing portion, a slit groove is formed, where the resection saw may be inserted, to form the resection pathway of the resection saw during the resection surgery.

16. The guide for mandibular resection surgery, according to claim 9,
wherein an inner side of the second guide plate is formed to be dented towards the inside, in consideration of a thickness of gum.

17. The guide for mandibular resection surgery, according to claim 16,
wherein the second guide plate from which the second fixing portion extends and the third fixing portion are arranged in one row in a vertical direction, and
the second fixing portion, the second guide plate, the third fixing portion, and the middle portion of the first guide plate from which the third fixing portion extends, arranged in one row, are formed of a hard material, integrally.

18. The guide for mandibular resection surgery, according to claim 8,
wherein a middle portion of a rear end of the first guide plate is made of a hard material, to prevent deformation from occurring due to self-weight.

19. The guide for mandibular resection surgery, according to claim 8,
wherein a metal rod is inserted into a middle portion of a rear end of the first guide plate to prevent deformation from occurring due to self-weight.

20. The guide for mandibular resection surgery, according to claim 1,
wherein a shape design of the guide plate is customized to a patient in consideration of a shape of the mandible of the patient and distribution of a neural tube inside the mandible, based on 3-dimensional image information on the mandible of the patient, so as not to damage the neural tube during the mandibular resection surgery, and
a shape design of the fixing portion is customized to the patient and manufactured such that a position of the guide plate can be stably fixed according to the shape of the mandible of the patient.

21. The guide for mandibular resection surgery, according to claim 1,
that is manufactured integrally using a 3-dimensional printer.
